# EUROPEAN PATENT SPECIFICATION

(11) **EP 3 246 308 B1**
(45) Date of publication and mention of the grant of the patent: **11.03.2020**
(21) Application number: 16737379.4
(22) Date of filing: 13.01.2016
(51) Int. Cl.: C07C 213/10, C07C 213/02, C07C 219/32

(54) **METHOD FOR PRODUCING AROMATIC AMINE COMPOUND**
VERFAHREN ZUR HERSTELLUNG VON AROMATISCHER AMINVERBINDUNG
PROCÉDÉ DE FABRICATION DE COMPOSÉ D'AMINE AROMATIQUE

(30) Priority: 13.01.2015 JP 2015004365
(43) Date of publication of application: 22.11.2017
(73) Proprietor: Nissan Chemical Corporation, Tokyo (JP)
(72) Inventor: NAGAO, Masato, Funabashi-shi Chiba 274-0052 (JP)
(74) Representative: Blodig, Wolfgang
(86) International application number: PCT/JP2016/050849
(87) International publication number: WO 2016/114308

(56) References cited:
- WO-A1-98/52990
- WO-A1-2008/096145
- WO-A1-2014/208609
- JP-A- H02 275 857
- JP-A- 2002 506 466
- JP-A- 2010 518 060

## Description

### TECHNICAL FIELD

The present invention relates to a method for producing an aromatic amine compound.

### BACKGROUND ART

As the method for removing a residue of a Sn compound used as a catalyst for reducing an aromatic nitro compound as a starting material with SnCl₂ (stannous chloride) to produce an aromatic amine compound, the following methods have already been known.

Patent Document 1 and Non-Patent Document 1 disclose the method of adding a sodium hydrogen carbonate aqueous solution to a reaction mixture for neutralization after a reaction and filtering precipitates derived from Sn.

Patent Document 2 discloses the method of distilling off a solvent after a reaction, adding a sodium hydroxide aqueous solution to the residue and adjusting pH to about 12 to 14, followed by extracting a product with ethyl acetate.

Patent Document 3 discloses the method of adding sodium hydroxide to a reaction liquid so as to be strong basic, followed by extracting a product with diethyl ether.

Patent Document 4 discloses the method of adding a potassium hydroxide aqueous solution to a reaction liquid which is an ethyl acetate solution and extracting a product with ethyl acetate, followed by purifying the product by silica gel column chromatography.

### PRIOR ART DOCUMENTS

### PATENT DOCUMENTS

Patent Document 1: WO2013/099804
Patent Document 2: JP-A-2012-526061
Patent Document 3: JP-A-2010-530405
Patent Document 4: JP-A-2008-526910

### NON-PATENT DOCUMENT

Non-Patent Document 1: J. Org. Chem. year 2005, Vol. 70, pages 63 to 78. WO 98/52990 discloses fluorinated amine products.
WO2008/096145 A1 discloses a process for preparing anagrelide.

### DISCLOSURE OF INVENTION

### TECHNICAL PROBLEM

It is an object of the present invention to provide a novel method for simply and efficiently removing a Sn compound to be formed at the time of reducing an aromatic nitro compound with SnCl₂ (stannous chloride) to produce an aromatic amine compound.

### SOLUTION TO PROBLEM

The present inventors have extensively studied in order to accomplish the above object, and as a result, they have accomplished the present invention. That is, the present invention provides the following.
1. A method for producing an aromatic amine compound represented by the formula 2, which comprises reducing an aromatic nitro compound represented by the formula 1 with stannous chloride in accordance with the following reaction formula (1),
   wherein a reaction liquid obtained by reducing the aromatic nitro compound represented by the formula 1, is brought into contact with a mixed liquid containing a compound having a benzene ring and a potassium hydroxide aqueous solution to carry out liquid separation, and from the obtained organic layer, the aromatic amine compound represented by the formula 2 is obtained: 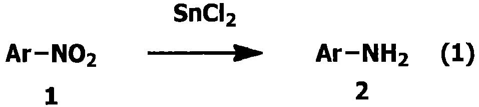 where Ar is an aromatic group.
2. The method according to the above 1, wherein the compound represented by the formula 1 is an aromatic dinitro compound represented by the formula 3:

   **O₂N-Ar²·NO₂** **3**

   where Ar² is an aromatic group excluding Ar.
3. The method according to the above 1, wherein the compound represented by the formula 1 is an aromatic dinitro compound represented by the formula 4: 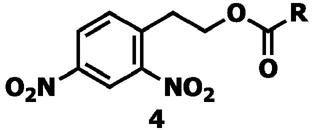 where R is an organic group inert to the reduction with stannous chloride.
4. The method according to the above 1, wherein the compound represented by the formula 1 is an aromatic dinitro compound represented by the formula 5: 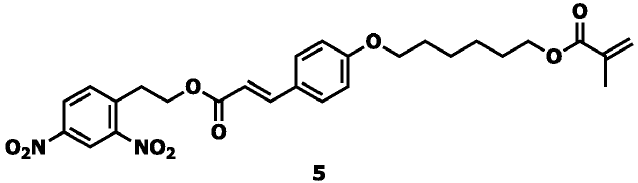
5. The method according to any one of the above 1 to 4, wherein the concentration of the potassium hydroxide aqueous solution is from 1 to 50 mass%.
6. The method according to any one of the above 1 to 5, wherein the amount of potassium hydroxide is at least 3 times by mol per 1 mol of tin atoms.
7. The method according to any one of the above 1 to 6, wherein the ratio in amount to be used of the potassium hydroxide aqueous solution to the compound having a benzene ring is from 50:1 to 1:50 by mass ratio.
8. The method according to any one of the above 1 to 7, wherein the temperature at the liquid separation step is from 10 to 80°C.
9. The method according to any one of the above 1 to 8, wherein the reaction liquid is added into the mixed liquid containing a compound having a benzene ring and a potassium hydroxide aqueous solution.
10. The method according to any one of the above 1 to 9, wherein the compound having a benzene ring has a boiling point of from 80 to 170°C.
11. The method according to any one of the above 1 to 10, wherein the compound having a benzene ring is toluene.
12. The method according to any one of the above 1 to 11, wherein tetrahydrofuran is used as a reaction solvent.
13. The method according to any one of the above 1 to 12, wherein water is used as a reaction solvent.

### ADVANTAGEOUS EFFECTS OF INVENTION

According to the present invention, in the method for producing an aromatic amine compound by reducing an aromatic nitro compound as a starting material with SnCl₂, a Sn compound in a reaction liquid can be simply and efficiently removed, whereby a highly pure aromatic amine compound can be obtained.

### DESCRIPTION OF EMBODIMENTS

In the method of the present invention, in accordance with the following reaction formula (1), the aromatic nitro compound represented by the formula 1 is reduced by means of stannous chloride to produce the aromatic amine compound represented by the formula 2.

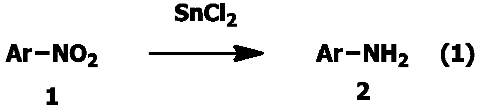

In the formula 1, Ar is an aromatic group, and the number of NO₂ groups is at least one and may be two or more, however, it is preferably from 1 to 4, more preferably from 1 to 3, particularly preferably 1 or 2. Needless to say, depending on the number of NO₂ groups, the valence of the aromatic groups as Ar varies. The aromatic ring in the aromatic group may be substituted by an alkyl group or a halogen atom.

As examples of the nitro compound represented by the formula 1 which is used in the present invention, the following formulae 1-1 to 1-53 may be mentioned.

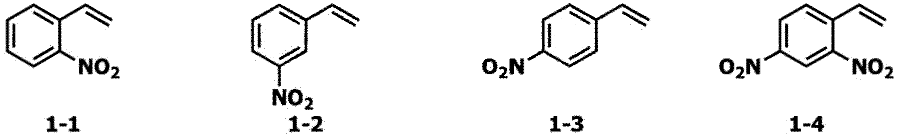

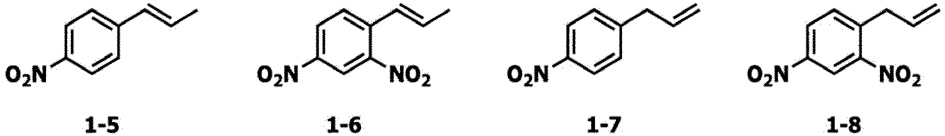

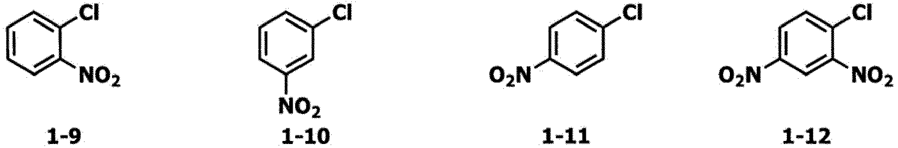

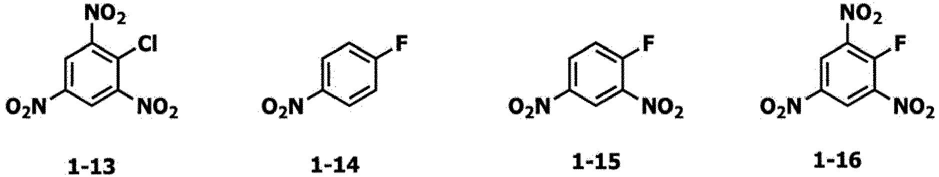

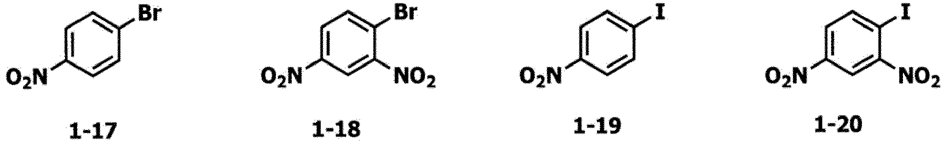

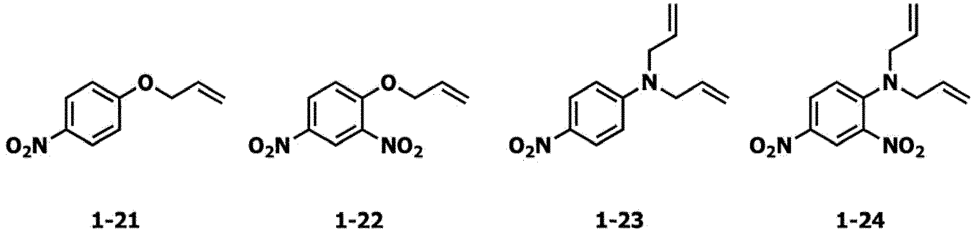

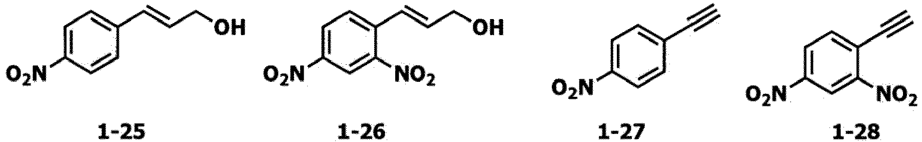

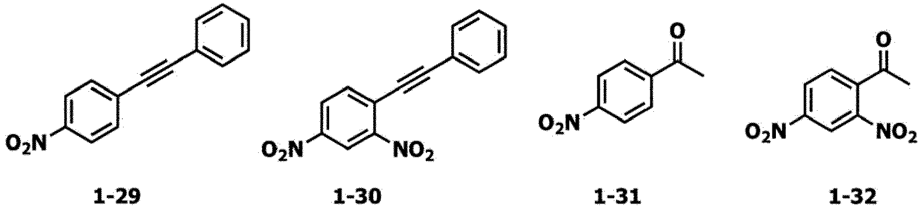

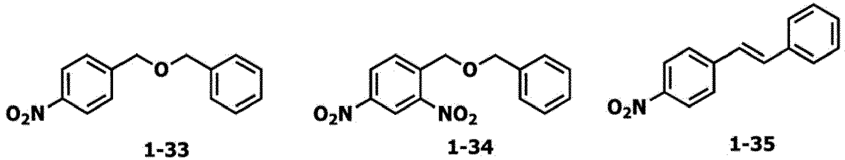

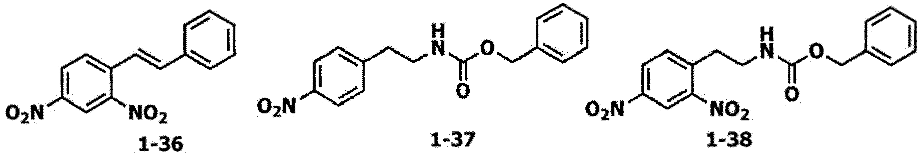

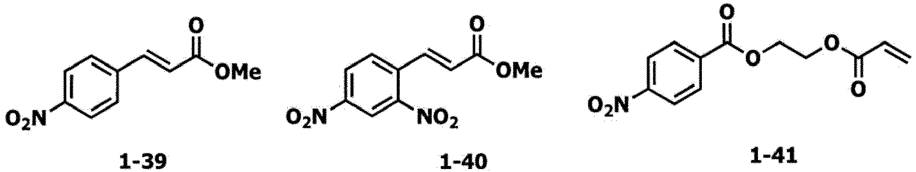

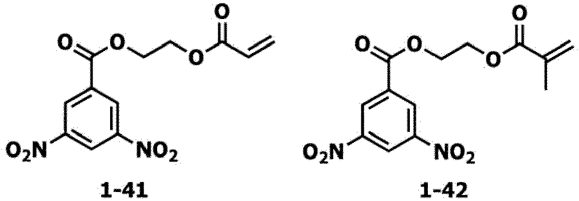

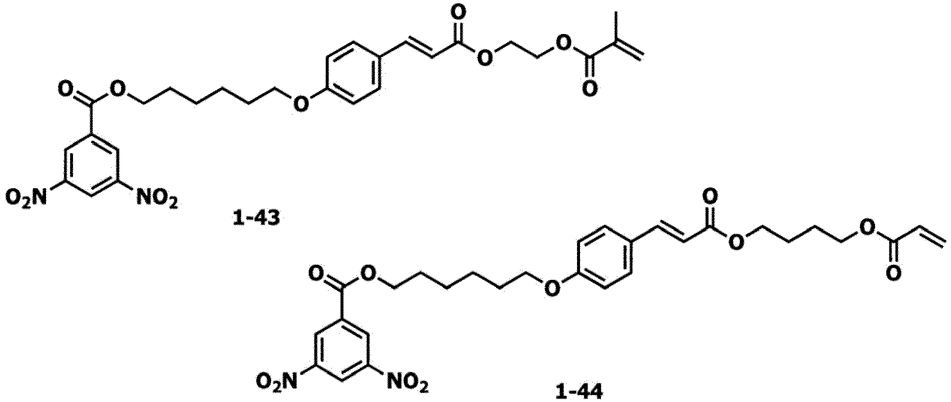

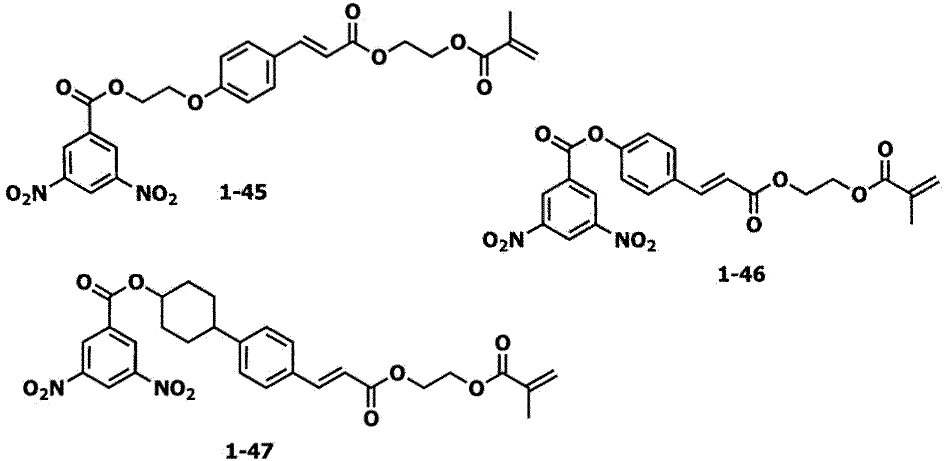

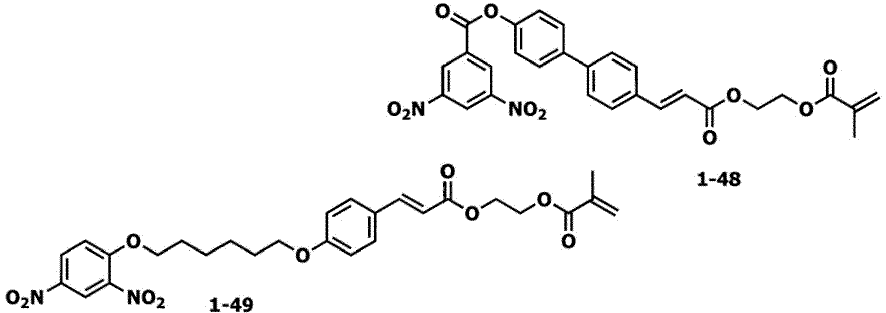

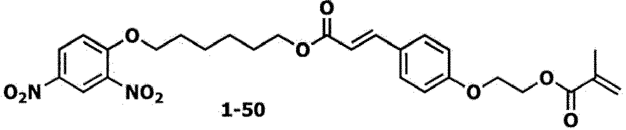

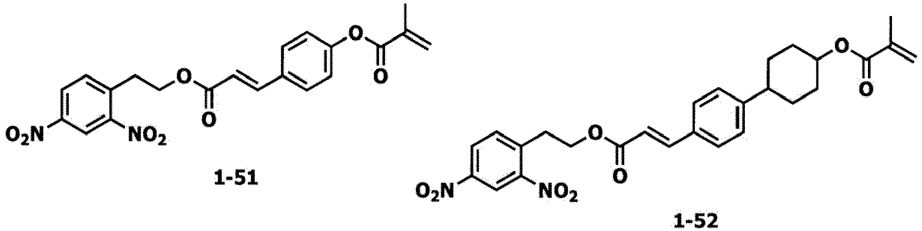

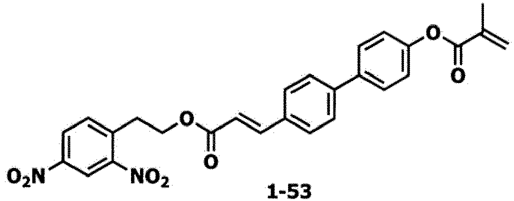

Further, as examples of the nitro compound represented by the formula 1 which is used in the present invention, the dinitro compounds mentioned in WO2007/071091, p.81, the compounds (where diamino shall be read as dinitro) mentioned in WO2007/071091, p.82 to p.88, the dinitro compound mentioned in WO2007/071091, p.88, the compounds (where diamino shall be read as dinitro) mentioned in WO2007/071091, p.89 to p.92, the dinitro compound mentioned in WO2007/071091, p.93, the compounds (where diamino shall be read as dinitro) mentioned in WO2007/071091, p.94 to p.99, the dinitro compounds mentioned in WO2007/071091, p.101, the compounds (where amino shall be read as nitro) mentioned in WO2007/071091, p.102 to p.104, the dinitro compounds mentioned in WO2007/071091, p.104, the dinitro compounds mentioned in WO2007/071091, p.107, the compounds (where diamino shall be read as dinitro) mentioned in WO2007/071091, p.109 to p.110, the dinitro compounds mentioned in WO2007/071091, p.111, the compounds (where diamino shall be read as dinitro) mentioned in WO2007/071091, p.112 to p.115, the dinitro compounds mentioned in WO2008/145225, p.87 and p.88, the compounds (where diamino shall be read as dinitro) mentioned in WO2008/145225, p.89 to p.93, the dinitro compounds mentioned in WO2008/145225, p.93, the compounds (where diamino shall be read as dinitro) mentioned in WO2008/145225, p.95 to p.106, the dinitro compounds mentioned in WO2008/145225, p.106, the compounds (where diamino shall be read as dinitro) mentioned in WO2008/145225, p.108 to p.135, the dinitro compounds mentioned in WO2008/145225, p.137, the compounds (where amino shall be read as nitro) mentioned in WO2008/145225, p.139 to p.141, the dinitro compounds mentioned in WO2008/145225, p.143, the dinitro compounds mentioned in WO2008/145225, p.145, the compounds (where amino shall be read as nitro) mentioned in WO2008/145225, p.146 to p.153 and the dinitro compounds mentioned in WO2013/099804, p.51, p.55, p.58, p.60, p.63, p.65, p.68, p.71 and p.74 may be mentioned.

In a case where the number of NO₂ groups in the nitro compound represented by the formula 1 is 2, the nitro compound is represented by the following formula 3.

**O₂N-Ar²-NO₂** **3**

The nitro compound represented by the formula 3 may, for example, be a dinitro compound represented by the following formula 4. The formula 4 has two NO₂ groups at meta, however, it may have two NO₂ groups at ortho or para and preferably at meta.

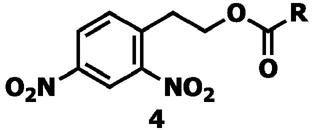

In the formula 4, R is an organic group inert to the reduction with stannous chloride and may, for example, be an alkyl group such as a methyl group, an ethyl group, a n-propyl group, an i-propyl group, a n-butyl group, an i-butyl group, a s-butyl group, a t-butyl group, a n-hexyl group, a n-octyl group or a n-decyl group; a cycloalkyl group such as a cyclopentyl group or a cyclohexyl group; a bicycloalkyl group such as a bicyclohexyl group; an alkenyl group such as a vinyl group, an 1-propenyl group, a 2-propenyl group, an isopropenyl group, a 1-methyl-2-propenyl group, a 1 or 2 or 3-butenyl group, a 1-hexenyl group, an α-styryl group or a β-styryl group; an aryl group such as a phenyl group, a xylyl group, tolyl group, a biphenyl group or a naphthyl group; or an aralkyl group such as a benzyl group, a phenyl ethyl group or phenyl cyclohexyl group. Among them, the vinyl group or the β-styryl group is preferred.

Further, a part of or all of hydrogen atoms in such organic groups may be substituted by halogen atoms, hydroxy groups, thiol groups, phosphoric acid ester groups, ester groups, carboxy groups, phosphoric acid groups, thioester groups, amide groups, organooxy groups, organosilyl groups, organothio groups, organoamino groups, carbamic acid ester groups, acyl groups, alkyl groups, cycloalkyl groups, bicycloalkyl groups, alkenyl groups, aryl groups or aralkyl groups. Further, such a group may be a ring structure.

The nitro compound represented by the formula 4 may, for example, be a dinitro compound represented by the following formula 5.

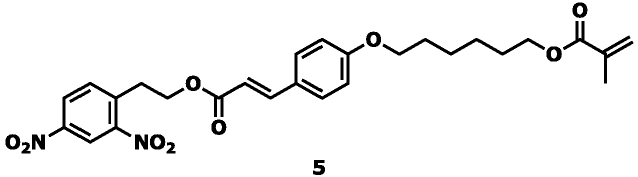

The above reduction reaction may be carried out by using or not using a reaction solvent, however, the reduction reaction is preferably carried out by using a reaction solvent.

The reaction solvent is not particularly restricted, so long as it is one inert to the reaction. For example, a hydrocarbon such as hexane, cyclohexane, benzene or toluene; a halogenated hydrocarbon such as carbon tetrachloride, chloroform or 1,2-dichloroethane; an alcohol such as methanol, ethanol or isopropyl alcohol; an ether such as diethyl ether, diisopropyl ether, 1,4-dioxane or tetrahydrofuran; a ketone such as acetone, methyl ethyl ketone or methyl isobutyl ketone; a nitrile such as acetonitrile or propionitrile; a carboxylic acid ester such as ethylacetate or ethylpropionate; a nitrogen-containing non-protic polar solvent such as N,N-dimethylformamide, N,N-dimethylacetamide, N-methyl-2-pyrrolidone or 1,3-dimethyl-2-imidazolidinone; a sulfur-containing non-protic polar solvent such as dimethylsulfoxide or sulfolane; a pyridine such as pyridine or picoline; or water may be mentioned. Such a solvent may be used alone, or two or more of such solvents may be used in combination. The reaction solvent is preferably a mixed solvent of a solvent selected from tetrahydrofuran and 1,4-dioxane and water, and further preferably a mixed solvent of tetrahydrofuran and water.

In a case where a mixed solvent of water and an organic solvent is used, the mixing ratio of the organic solvent to water is from 1:50 to 50:1, preferably 1:10 to 10:1 by mass ratio.

The amount (concentration) of the reaction solvent to be used is not particularly restricted, however, the reaction solvent may be used in an amount of from 1 to 100 times by mass, per 1 part by mass of the aromatic nitro compound represented by the formula 1. The amount is preferably from 1.5 to 50 times by mass, further preferably from 2.5 to 10 times by mass.

The reaction temperature is not particularly restricted. For example, the reaction temperature is from -90 to 200°C, preferably from 0 to 150°C, further preferably from 10 to 120°C. The reaction time is usually from 0.05 to 200 hours, preferably from 0.5 to 100 hours.

The amount of stannous chloride to be used in the reduction reaction is not particularly restricted. However, the amount is preferably from 1 to 10 times by mol, further preferably 2 to 5 times by mol, per 1 mol of the nitro group in the aromatic nitro compound represented by the formula 1. For example, in a case where the aromatic nitro compound represented by the formula 1 has two nitro groups, the amount of stannous chloride to be used is preferably 2 to 20 times by mol, further preferably from 4 to 10 times by mol, per the aromatic nitro compound.

The specific reduction reaction is preferably carried out as described below. That is, an aromatic nitro compound, stannous chloride and a reaction solvent e.g. tetrahydrofuran and water as starting materials are charged in a reactor, followed by stirring at preferably from 10 to 120°C for preferably from 1 to 20 hours.

As the order of charging the starting materials, in order to prevent side reactions of reaction intermediates, the method of dividedly adding the aromatic nitro compound or adding the reaction solvent having the aromatic nitro compound dissolved therein into the mixture of stannous chloride and the reaction solvent is preferred. The termination of the reaction can be confirmed by thin layer chromatography, high performance liquid chromatography or the like.

As described above, the aromatic nitro compound represented by the formula 1 is reduced to form the aromatic amine represented by the formula 2, whereby a reaction liquid containing the aromatic amine, unreacted starting materials and a Sn compound is obtained.

Then, the obtained reaction liquid is subjected to a liquid separation step in which a mixed liquid containing a compound having a benzene ring and a potassium hydroxide aqueous solution is used. The compound having a benzene ring has a boiling point of preferably from 80 to 170°C, more preferably from 80 to 150°C so as to be easily concentrated after the liquid separation. As the compound having a benzene ring, benzene, toluene, m-xylene, o-xylene, p-xylene, mesitylene, anisole, chlorobenzene or o-dichlorobenzene may be mentioned. Toluene is particularly preferred, since the toxicity is relatively low, and the distillation and drying can be easily carried out.

For example, in a case where toluene is used as the compound having a benzene ring in the liquid separation step of separating the Sn compound from the reaction liquid to obtain the desired product, the following four methods may be mentioned.
(Method 1): A potassium hydroxide aqueous solution is added to a reaction liquid, followed by adding toluene for liquid separation, and the desired product is obtained from the organic layer.
(Method 2): Toluene is added to a reaction liquid, the mixture is washed with a potassium hydroxide aqueous solution to carry out liquid separation, and the desired product is obtained from the organic layer.
(Method 3): A reaction liquid is added in a container in which toluene and a potassium hydroxide aqueous solution are added to carry out liquid separation, and the desired product is obtained from the organic layer.
(Method 4): Toluene and potassium hydroxide aqueous solution are added in a reaction liquid at one time to carry out liquid separation, and the desired product is obtained from the organic layer.

In the present invention, any method may be used, however, the above (method 3) is particularly preferred, since when mixing the potassium hydroxide aqueous solution, the temperature can be controlled at constant, the stirring failure due to the precipitation of the desired compound and the Sn compound during mixing will not be caused, and after mixing, the Sn compound will not be precipitated, and thereby the interface of two layers can be clearly distinguished.

The amount of potassium hydroxide in the potassium hydroxide aqueous solution used in the liquid separation step is preferably an amount such that the Sn compound in the reaction liquid is made to be potassium hexahydrostannate having a high solubility, and the amount is preferably at least 3 times by mol, more preferably from 3 to 10 times by mol, particularly preferably from 3 to 6 times by mol, per 1 mol of tin atoms.

The concentration of the potassium hydroxide aqueous solution to be used is not particularly restricted, however, so long as the desired compound is not decomposed, the higher the concentration is, it is more preferred, since the treatment can be carried out with a small amount of the potassium hydroxide aqueous solution. If the desired product is decomposed, the treatment can be carried out by lowering the concentration of the potassium hydroxide aqueous solution. Thus, the concentration of the potassium hydroxide aqueous solution is preferably from 1 to 50 mass%, more preferably from 10 to 50 mass%.

Further, the ratio in amount of the compound having a benzene ring to the potassium hydroxide aqueous solution is preferably from 100:1 to 1:100, more preferably from 75:1 to 1:75, particularly preferably from 50:1 to 1:50 by mass ratio, from the viewpoint of easily forming the interface at the time of the liquid-separation, and improving the volume efficiency.

The temperature in the liquid separation is not particularly restricted, so long as the desired product is not decomposed and is dissolved. The temperature is preferably from 10 to 80°C and from the viewpoint of easily controlling the temperature, more preferably from 10 to 60°C. The time spent for the liquid separation is from 0.01 to 4 hours, preferably from 0.02 to 1 hour.

A layer of the compound having a benzene ring, which is an organic layer to be obtained by the above liquid separation, contains an aromatic amine compound which is the desired compound, and the organic layer may be concentrated as it is, and the desired product may be separated and recovered. Further, a poor solvent may be added to the organic layer to crystallize the desired product, and thereby the aromatic amine compound can be recovered by the crystallization. In such a case, as the poor solvent, a hydrocarbon such as hexane, heptane or cyclohexane, an alcohol such as ethanol, methanol or i-propyl alcohol or the like is preferably used. Further, a purification treatment such as recrystallization may be carried out in order to further increase the purity of the desired product.

On the other hand, the water layer formed by the liquid separation contains potassium hexahydrostannate having a high solubility which is derived from stannous chloride used in the reduction reaction, and the water layer may be discarded as it is, however, as the case requires, the tin compound may be recovered.

### EXAMPLES

Now, the present invention will be described in further detail with reference to Examples. However, it should be understood that the present invention is by no means restricted thereto. Further, analyzing devices and analyzing conditions employed in Examples are mentioned below.

### HPLC Analysis

Device: LC-20A system (manufactured by Shimadzu Corporation)
Column: Inertsil ODS-3 (4.6 mm Φ × 250 mm, manufactured by GL Sciences Inc.)
Detector: UV detector (wavelength: 254 nm)
Eluent: acetonitrile/0.1 wt% acetic acid aqueous solution (70/30, v/v)

### EXAMPLE 1

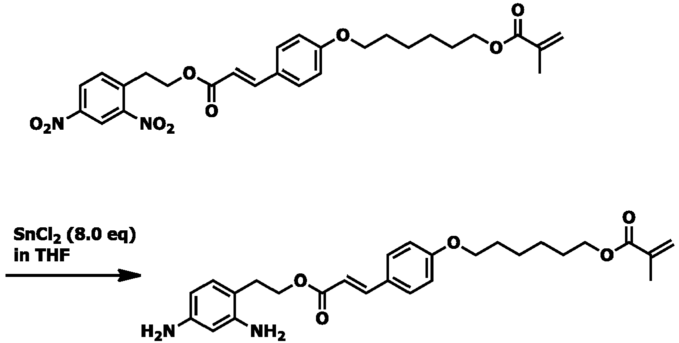

Stannous chloride (43.2 g, 228 mmol) was dissolved in a mixed reaction solvent of THF (tetrahydrofuran) (45.0 g) and water (75.0 g), and a solution having (E)-4-(6-(methacryloyloxy)hexyloxy) cinnamic acid (2-(2,4-dinitrophenyl)ethyl)ester (15.0 g, 28.5 mmol) dissolved in THF (27.0 g) was dropwise added thereto at from 18 to 25°C over 1 hour. Then, the mixture was stirred for 25 hours to obtain a reaction mixture containing (E)-4-(6-(methacryloyloxy)hexyloxy) cinnamic acid (2-(2,4-diaminophenyl)ethyl)ester.

Then, the above reaction mixture was dropwise added to a mixed solution of toluene (75.0 g) and a 40 mass% potassium hydroxide aqueous solution (105 g). At that time, precipitates were not formed during the dropwise addition, and stirring could be successfully carried out. Then, the mixture was stirred for 10 minutes, the water layer was discarded, and a 6 mass% potassium hydroxide aqueous solution (75 g) was added to the obtained organic layer, followed by stirring. Then, the water layer was discarded. Then, a treatment of adding water (75 g), stirring and discarding a water layer was repeated 5 times. Then, the organic layer was concentrated, and toluene was added so that the total amount would be 90.6 g, and precipitates were dissolved at 50°C. Then, the mixture was cooled to 0°C, and a precipitated solid was filtered and dried (40°C) to obtain the desired product of (E)-4-(6-methacryloyloxy)hexyloxy) cinnamic acid (2-(2,4-dinitrophenyl)ethyl)ester (pale brown solid, obtained amount: 11.0 g, yield: 83%, Sn content: 70 ppm).

Further, the above obtained solid (1.00 g) was dissolved in toluene (7.00 g) at 50°C, and 0.1 g of activated carbon (Special Shirasagi, manufactured by Japan EnviroChemicals, Ltd.) was added thereto, followed by stirring for 30 minutes. Then, the activated carbon was filtered out, and the filtrate was concentration. As a result, the Sn content in the desired product was lower than 1 ppm.

### COMPARATIVE EXAMPLE 1

The experiment was carried out in the same manner as in Ex. 1, except that the amount of (E)-4-(6-(methacryloyloxy)hexyloxy) cinnamic acid (2-(2,4-dinitrophenyl)ethyl)ester as the starting material was changed to 1.0 g, and the proportions of the other starting materials were the same as those in Example 1. 5 g of ethylacetate was added to the reaction mixture containing the desired product of (E)-4-(6-(methacryloyloxy)hexyloxy) cinnamic acid (2-(2,4-diaminophenyl)ethyl)ester, and then sodium hydrogen carbonate (2.4 g) was added thereto. As a result, a large amount of white precipitates were formed, and thereby stirring could not be carried out.

### COMPARATIVE EXAMPLE 2

The reaction mixture containing (E)-4-(6-(methacryloyloxy)hexyloxy) cinnamic acid (2-(2,4-diaminophenyl)ethyl)ester was obtained in the same manner as in Comparative Example 1. 7.2 g which was the amount of one third of the reaction mixture was taken out, 3.3 g of ethylacetate was added thereto, and further a 40 mass% sodium hydroxide aqueous solution was dropwise added thereto. When 1.3 g of the 40 mass% sodium hydroxide aqueous solution was dropwise added, a large amount of white precipitates were formed, and stirring could not be carried out. Further, 1.9 g of 40 mass% sodium hydroxide aqueous solution was dropwise added, and the precipitates were dissolved. However, a mixture was allowed to stand for the next three days, and precipitates were formed.

### COMPARATIVE EXAMPLE 3

A reaction mixture containing (E)-4-(6-(methacryloyloxy)hexyloxy) cinnamic acid (2-(2,4-diaminophenyl)ethyl)ester was obtained in the same manner as in Comparative Example 1. 5.0 g of ethylacetate was added to the reaction mixture, and further a 40 mass% potassium hydroxide aqueous solution (7.0 g) was dropwise added. During the dropwise addition, white precipitates where formed, and the stirring property deteriorated. However, after the completion of dropwise adding the total amount, the precipitates were dissolved. Then, after allowing to stand for a few days, precipitates formed again.

### INDUSTRIAL APPLICABILITY

In the method of the present invention for producing an aromatic amine by reducing an aromatic nitro compound by means of stannous chloride, a Sn compound can be simply and efficiently removed, and a highly pure aromatic amine compound can be obtained. Thus, the method of the present invention is industrially useful.

## Claims

1. A method for producing an aromatic amine compound represented by the formula 2, which comprises reducing an aromatic nitro compound represented by the formula 1 with stannous chloride in accordance with the following reaction formula (1),
wherein a reaction liquid obtained by reducing the aromatic nitro compound represented by the formula 1, is brought into contact with a mixed liquid containing a compound having a benzene ring and a potassium hydroxide aqueous solution to carry out liquid separation, and from the obtained organic layer, the aromatic amine compound represented by the formula 2 is obtained: 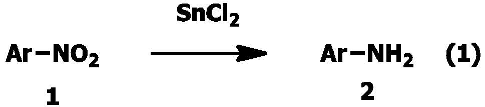 where Ar is an aromatic group.

2. The method according to Claim 1, wherein the compound represented by the formula 1 is an aromatic dinitro compound represented by the formula 3:
**O₂N-Ar²·NO₂** **3**
where Ar² is an aromatic group excluding Ar.

3. The method according to Claim 1, wherein the compound represented by the formula 1 is an aromatic dinitro compound represented by the formula 4: 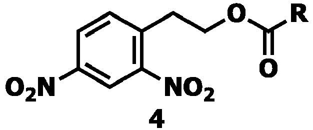 where R is an organic group inert to the reduction with stannous chloride.

4. The method according to Claim 1, wherein the compound represented by the formula 1 is an aromatic dinitro compound represented by the formula 5:

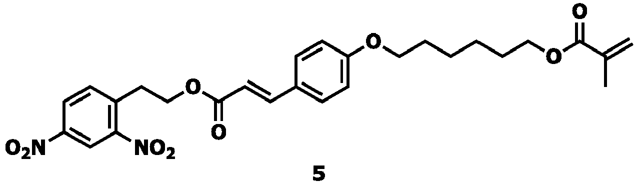

5. The method according to any one of Claims 1 to 4, wherein the concentration of the potassium hydroxide aqueous solution is from 1 to 50 mass%.

6. The method according to any one of Claims 1 to 5, wherein the amount of potassium hydroxide is at least 3 times by mol per 1 mol of tin atoms.

7. The method according to any one of Claims 1 to 6, wherein the ratio in amount to be used of the potassium hydroxide aqueous solution to the compound having a benzene ring is from 50:1 to 1:50 by mass ratio.

8. The method according to any one of Claims 1 to 7, wherein the temperature at the liquid separation step is from 10 to 80°C.

9. The method according to any one of Claims 1 to 8, wherein the reaction liquid is added into the mixed liquid containing a compound having a benzene ring and a potassium hydroxide aqueous solution.

10. The method according to any one of Claims 1 to 9, wherein the compound having a benzene ring has a boiling point of from 80 to 170°C.

11. The method according to any one of Claims 1 to 10, wherein the compound having a benzene ring is toluene.

12. The method according to any one of Claims 1 to 11, wherein tetrahydrofuran is used as a reaction solvent.

13. The method according to any one of Claims 1 to 12, wherein water is used as a reaction solvent.

## Patentansprüche

1. Verfahren zur Herstellung einer aromatischen Aminverbindung der Formel 2, welches das Reduzieren einer aromatischen Nitroverbindung der Formel 1 mit Zinn-II-Chlorid in Übereinstimmung mit der folgenden Reaktionsformel (1) umfasst, wobei die Reaktionsflüssigkeit, die durch Reduzieren der aromatischen Nitroverbindung der Formel 1 erhalten wird, in Kontakt mit einer gemischten Flüssigkeit gebracht wird, die eine Verbindung mit einem Benzolring und eine wässrige Kaliumhydroxidlösung enthält, um eine Flüssigtrennung durchzuführen, und aus der erhaltenen organischen Schicht die aromatische Aminverbindung der Formel 2 erhalten wird: 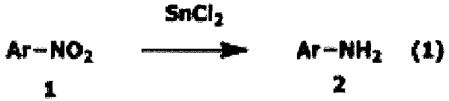 wobei Ar für eine aromatische Gruppe steht.

2. Verfahren nach Anspruch 1, wobei die Verbindung der Formel 1 eine aromatische Dinitroverbindung der Formel 3 ist:
**O₂NAr²-NO₂** **3**
wobei Ar² für eine aromatische Gruppe steht, wobei Ar ausgeschlossen ist.

3. Verfahren nach Anspruch 1, wobei die Verbindung der Formel 1 eine aromatische Dinitroverbindung der Formel 4 ist: 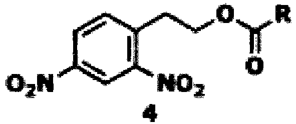 wobei R für eine organische Gruppe steht, die gegenüber der Reduktion mit Zinn-II-Chlorid inert ist.

4. Verfahren nach Anspruch 1, wobei die Verbindung der Formel 1 eine aromatische Dinitroverbindung der Formel 5 ist:

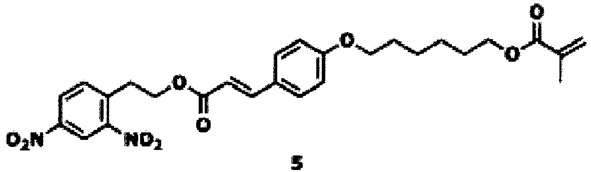

5. Verfahren nach einem der Ansprüche 1 bis 4, wobei die Konzentration der wässrigen Kaliumhydroxidlösung von 1 bis 50 Masseprozent beträgt.

6. Verfahren nach einem der Ansprüche 1 bis 5, wobei die Menge an Kaliumhydroxid wenigstens 3-fach nach Mol pro 1 Mol Zinnatome beträgt.

7. Verfahren nach einem der Ansprüche 1 bis 6, wobei das zu verwendende Mengenverhältnis der wässrigen Kaliumhydroxidlösung zu der Verbindung mit dem Benzolring von 50:1 bis 1:50 nach Massenverhältnis beträgt.

8. Verfahren nach einem der Ansprüche 1 bis 7, wobei die Temperatur in dem Schritt der Flüssigtrennung von 10 bis 80°C beträgt.

9. Verfahren nach einem der Ansprüche 1 bis 8, wobei die Reaktionsflüssigkeit in die gemischte Flüssigkeit, die die Verbindung mit dem Benzolring und die wässrige Kaliumhydroxidlösung enthält, gegeben wird.

10. Verfahren nach einem der Ansprüche 1 bis 9, wobei die Verbindung mit dem Benzolring einen Siedepunkt von 80 bis 170°C aufweist.

11. Verfahren nach einem der Ansprüche 1 bis 10, wobei die Verbindung mit dem Benzolring Toluol ist.

12. Verfahren nach einem der Ansprüche 1 bis 11, wobei Tetrahydrofuran als Reaktionslösungsmittel verwendet wird.

13. Verfahren nach einem der Ansprüche 1 bis 12, wobei Wasser als Reaktionslösungsmittel verwendet wird.

## Revendications

1. Procédé pour produire un composé amine aromatique représenté par la formule 2, qui comprend la réduction d'un composé nitro aromatique représenté par la formule 1 avec du chlorure stanneux conformément à la formule réactionnelle (1) qui suit,
dans lequel le liquide réactionnel obtenu par réduction du composé nitro aromatique représenté par la formule 1 est porté en contact avec un liquide mixte contenant un composé ayant un cycle benzène et une solution aqueuse d'hydroxyde de potassium pour que s'effectue une séparation des liquides et, à partir de la phase organique obtenue, le composé amine aromatique représenté par la formule 2 est obtenu : 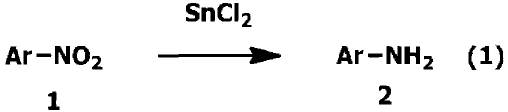 où Ar est un groupe aromatique.

2. Procédé selon la revendication 1, dans lequel le composé représenté par la formule 1 est un composé dinitro aromatique représenté par la formule 3 :
**O₂N-Ar²·NO₂** **3**
où Ar² est un groupe aromatique à l'exclusion d'Ar.

3. Procédé selon la revendication 1, dans lequel le composé représenté par la formule 1 est un composé dinitro aromatique représenté par la formule 4 : 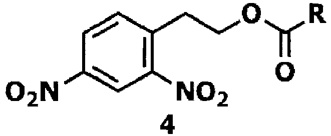 où R est un groupe organique inerte vis-à-vis de la réduction avec du chlorure stanneux.

4. Procédé selon la revendication 1, dans lequel le composé représenté par la formule 1 est un composé dinitro aromatique représenté par la formule 5 :

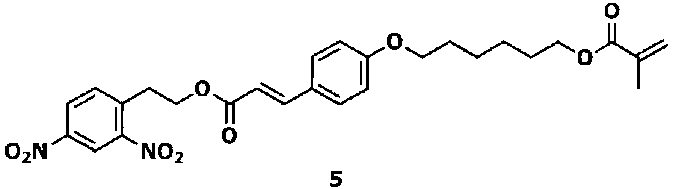

5. Procédé selon l'une quelconque des revendications 1 à 4, dans lequel la concentration de la solution aqueuse d'hydroxyde de potassium est de 1 à 50 % en masse.

6. Procédé selon l'une quelconque des revendications 1 à 5, dans lequel la quantité d'hydroxyde de potassium est d'au moins 3 moles par mole d'atomes d'étain.

7. Procédé selon l'une quelconque des revendications 1 à 6, dans lequel le rapport en quantité à utiliser de la solution aqueuse d'hydroxyde de potassium sur le composé ayant un cycle benzène est de 50/1 à 1/50 en rapport en masse.

8. Procédé selon l'une quelconque des revendications 1 à 7, dans lequel la température lors de l'étape de séparation des liquides est de 10 à 80°C.

9. Procédé selon l'une quelconque des revendications 1 à 8, dans lequel le liquide réactionnel est ajouté dans le liquide mixte contenant un composé ayant un cycle benzène et une solution aqueuse d'hydroxyde de potassium.

10. Procédé selon l'une quelconque des revendications 1 à 9, dans lequel le composé ayant un cycle benzène a un point d'ébullition de 80 à 170°C.

11. Procédé selon l'une quelconque des revendications 1 à 10, dans lequel le composé ayant un cycle benzène est le toluène.

12. Procédé selon l'une quelconque des revendications 1 à 11, dans lequel du tétrahydrofurane est utilisé en tant que solvant réactionnel.

13. Procédé selon l'une quelconque des revendications 1 à 12, dans lequel de l'eau est utilisée en tant que solvant réactionnel.
